# EUROPEAN PATENT APPLICATION

(11) **EP 1 637 159 A1**
(43) Date of publication of application: **22.03.2006**
(21) Application number: 04734385.0
(22) Date of filing: 21.05.2004
(51) Int. Cl.: A61K 39/395, A61P 37/06, A61P 1/16, A61P 11/06, A61P 19/02, A61P 3/10, A61P 9/10, A61P 11/10

(54) **IMMUNOCOMPETENT CELL ACTIVATION INHIBITOR AND USE THEREOF**

(30) Priority: 23.05.2003 JP 2003146188
(71) Applicant: Immuno-Biological Laboratories Co., Ltd., Fujioka-shi, Gunma 375-0005 (JP); Gene Techno Science Co., Ltd., Sapporo-shi, Hokkaido 062-8517 (JP)
(72) Inventor: KON, Shigeyuki, Sapporo-shi, Hokkaido 001-0017 (JP); UEDE, Toshimitsu, Sapporo-shi, Hokkaido 004-0835 (JP); DIAO, Hongyan, Sapporo-shi, Hokkaido 065-0012 (JP)
(74) Representative: Hartz, Nikolai
(86) International application number: PCT/JP2004/007321
(87) International publication number: WO 2004/103403

(57) **Abstract**

The invention relates to an immunocompetent cell activation inhibitor comprising an antibody against OPN or peptide fragment thereof; a therapeutic agent for diseases attributed to immunocompetent cell activation comprising this inhibitor as an active ingredient; and a method of treatment with the use of the therapeutic agent. The invention provides a medical agent capable of treating diseases attributed to immunocompetent cell activation, such as hepatopathy, asthma, arthritis, diabetes, lupus, multiple sclerosis, arteriosclerosis and lung fibrosis.

SEQUENCE LISTING

<110> Immuno-Biological Laboratories Co., Ltd.
G-in Techno Science Co., Ltd.

<120> Immunocompetent cell activation inhibitor and use thereof

<130> PF-040006-WO

<150> JP 2003-146188
<151> 2003-05-23

<160> 15

<170> PatentIn version 3.1

<210> 1
<211> 10
<212> PRT
<213> Artificial

<220>
<223> Description of Artificial Sequence:fragment peptide

<400> 1

Arg Gly Asp Ser Val Val Tyr Gly Leu Arg
1               5                   10

<210> 2
<211> 17
<212> PRT
<213> Artificial

<220>
<223> Description of Artificial Sequence:2k1 peptide

<400> 2

Val Asp Thr Tyr Asp Gly Arg Gly Asp Ser Val Val Tyr Gly Leu Arg
1               5                   10                  15

Ser

<210> 3
<211> 17
<212> PRT
<213> Artificial

<220>
<223> Description of Artificial Sequence:M5 peptide

<400> 3

Cys Val Asp Val Pro Asn Gly Arg Gly Asp Ser Leu Ala Tyr Gly Leu
1               5                   10                  15

Arg

<210> 4
<211> 20
<212> DNA
<213> Artificial

<220>
<223> primer

<400> 4
accacagtcc atgccatcac                                                    20

<210> 5
<211> 21
<212> DNA
<213> Artificial

<220>
<223> primer

<400> 5
tccaccaccc ctgttgctgt a                                                21

<210> 6
<211> 20
<212> DNA
<213> Artificial

<220>
<223> primer

<400> 6
tggaagctac ttaggctact                                                  20

<210> 7
<211> 20
<212> DNA
<213> Artificial

<220>
<223> primer

<400> 7
tcccacgact tcggtagtat                                                  20

<210> 8
<211> 30
<212> DNA
<213> Artificial

<220>
<223> primer

<400> 8
aaaggctgca gctgtcccac atggacgaag                                       30

<210> 9
<211> 30
<212> DNA

<213>  Artificial

<220>
<223>  primer

<400>  9
tttagagaga tattcttcac agcccccaaa                                        30


<210>  10
<211>  22
<212>  DNA
<213>  Artificial

<220>
<223>  primer

<400>  10
gaacaaaggc aaggctaact ga                                                22


<210>  11
<211>  22
<212>  DNA
<213>  Artificial

<220>
<223>  primer

<400>  11
aacataacaa catctgggca at                                                22


<210>  12
<211>  30
<212>  DNA
<213>  Artificial

<220>
<223>  primer

<400>  12
tagggatccc tcccggtgaa agtgactgat                                         30

<210> 13
<211> 29
<212> DNA
<213> Artificial

<220>
<223> primer

<400> 13
gtctcgagtt agttgacctc agaagatga 29

<210> 14
<211> 30
<212> DNA
<213> Artificial

<220>
<223> primer

<400> 14
aacctcgagt tacctcagtc cataagccaa 30

<210> 15
<211> 30
<212> DNA
<213> Artificial

<220>
<223> primer

<400> 15
cagggatcct caaagtctag gagtttccag 30

## Description

### TECHNICAL FIELD

The present invention relates to an immunocompetent cell activation inhibitor that comprises an antibody to osteopontin or peptide fragment thereof, and to its use.

### BACKGROUND ART

Immunocompetent cells such as B cells, macrophages, dendritic cells, T cells, NKT cells and neutrophils participate in biological immune systems.

However, it is suggested that abnormal activation of these immunocompetent cells may cause various diseases, for example, hepatopathy (Kaneko Y., et al., *J. Exp. Med.,* 2000, Jan. 3; 191(1); 105-14, Nakamura K., et al., *J. Hepatol. ,* 2001, Aug.; 35(2): 217-24, Tiegs G., et al., *J. Clin. Invest.,* 1992 Jul.; 90(1): 196-203), asthma (Akbari O., et al., *Nat. Med.,* 2003, May; 9(5): 582-8, LindenA., *Int. Arch. Allergy Immunol.,* 2001, Nov.; 126(3): 179-84, Corrigan CJ., *Chem. Immunol.,* 2000; 78: 39-49), arthritis (ChibaA., etal., *Infect. Immun.,* 2003, Oct.; 71(10): 5447-55, Nurieva RI., *J. Clin. Invest.,* 2003, Mar.; 111 (5) : 701-6, Taylor PC., *Curr. Opin. Pharmacol. ,* 2003, Jun.; 3(3) : 323-8), diabetes (Hong S., et al., *Nat. Med.,* 2001, Sep.; 7(9): 1052-6, Hahn HJ. , *Diabetes Metab. Rev.,* 1993, Dec.; 9(4) : 323-8), lupus (Zeng D., *J. Clin. Invest.,* 2003, Oct.; 112(8): 1211-22, Olive D., et al., *Crit. Rev. Ther. Drug Carrier Syst.,* 1993; 10(1): 29-63), multiple sclerosis (Singh AK., *J. Exp. Med.,* 2001, Dec. 17; 194(12): 1801-11, Maatta JA., et al., *J*. *Neuroimmunol . ,* 1998, Oct. 1; 90(2): 162-75, Milici AJ. , et al., *Lab. Invest.,* 1998, Oct.; 78(10): 1239-44), arteriosclerosis (Tupin E. et al., *J. Exp. Med.,* 2004, Feb. 2; 199(3) : 417-22), lung fibrosis (Keane MP., et al., *J. Immunol.,* 1999, May 1; 162(9): 5511-8, Hu H., et al., *J. Leukoc. Biol.,* 1993, Nov.; 54 (5) : 414-22).

Many diseases caused by activation of immunocompetent cells as above have heretofore been difficult to treat, since the mechanism of their outbreak is complicated.

Accordingly, an object of the invention is to provide a medical agent for treating the above-mentioned diseases.

### DISCLOSURE OF THE INVENTION

We, the applicant have previously found that an antibody to osteopontin (hereinafter referred to as "OPN"), which is an acidic calcium-binding glycoprotein much contained in bones, or to peptide fragment thereof (hereinafter referred to as "OPN-inhibitory antibody" ) is utilizable for treatment of autoimmune diseases, rheumatism and rheumatoid arthritis (WO02/081522 pamphlet and WO03/027151 pamphlet), and as a diagnostic agent for tuberculosis (JP-A 2003-294735), a preventive for deterioration of cords and bands (Japanese Patent Application No. 2003-389543) and a diagnostic agent for interstitial pneumonia (Japanese Patent Application No. 2003-194977), and have previously filed patent applications for them.

Further, we, the applicant have assiduously studied for the purpose of expanding the utilization of the OPN-inhibitory antibody, and have found that OPN or peptide fragment thereof participates in activation of the above-mentioned immunocompetent cells. Accordingly, we have found that, when OPN or peptide fragment thereof is inhibited by the OPN-inhibitory antibody, then the activation of immunocompetent cells can be inhibited.

In addition, we have found that utilization of the OPN-inhibitory antibody produced a therapeutic agent for diseases caused by activation of immunocompetent cells, and have completed the present invention.

Specifically, the invention is as follows:
(1) An immunocompetent cell activation inhibitor comprising an antibody to osteopontin or peptide fragment thereof.
(2) The immunocompetent cell activation inhibitor of (1), wherein the antibody to osteopontin or peptide fragment thereof is an antibody capable of inhibiting the binding between an integrin recognizing the site of amino acid sequence RGD and osteopontin or fragment thereof, and also inhibiting the binding between an integrin recognizing the site of amino acid sequence SVVYGLR and osteopontin or fragment thereof.
(3) The immunocompetent cell activation inhibitor of (1), wherein the osteopontin or peptide fragment thereof is an N-terminal fragment of osteopontin.
(4) The immunocompetent cell activation inhibitor of (1),
   wherein the osteopontin or peptide fragment thereof is a peptide that contains a peptide of the following (A):
   (A) RGDSVVYGLR
(5) The immunocompetent cell activation inhibitor of (1), wherein the osteopontin or peptide fragment thereof is a peptide that contains a peptide of the following (B):
   (B) VDTYDGRGDSVVYGLRS
(6) The immunocompetent cell activation inhibitor of any of (1) to (5), wherein the immunocompetent cells are NKT cells.
(7) The immunocompetent cell activation inhibitor of (6), wherein said inhibitor inhibits the IFN-γ production by NKT cells.
(8) The immunocompetent cell activation inhibitor of (6), wherein said inhibitor inhibits the MIP-2 production by NKT cells.
(9) The immunocompetent cell activation inhibitor of (6), wherein said inhibitor inhibits the IL-4 production by NKT cells.
(10) The immunocompetent cell activation inhibitor of any of (1) to (5), wherein the immunocompetent cells are neutrophils.
(11) The immunocompetent cell activation inhibitor of any of (1) to (5), wherein the immunocompetent cells are T cells.
(12) The immunocompetent cell activation inhibitor of (11), wherein T cells are CD4⁺ T cells.
(13) The immunocompetent cell activation inhibitor of any of (1) to (12), wherein said inhibitor inhibits Fas/FasL mediated cell injury.
(14) The immunocompetent cell activation inhibitor of any of (1) to (12), wherein said inhibitor inhibits neutrophil mediated cell injury.
(15) A therapeutic agent for diseases caused by activation of immunocompetent cells, comprising the immunocompetent cell activation inhibitor of any of (1) to (14) as the active ingredient.
(16) The therapeutic agent for diseases of (15), wherein the immunocompetent cells are one or more types of immunocompetent cells selected from NKT cells, neutrophils and T cells.
(17) The therapeutic agent for diseases of (15) or (16), wherein the diseases caused by activation of immunocompetent cells are selected from hepatitis, asthma, arthritis, diabetes, lupus, multiple sclerosis, arteriosclerosis and lung fibrosis.
(18) A therapeutic agent for hepatopathy, comprising the immunocompetent cell activation inhibitor of any of (1) to (14) as the active ingredient.
(19) The therapeutic agent for hepatopathy of (18), wherein the hepatopathy is viral hepatitis or drug-induced hapatitis.
(20) The therapeutic agent for hepatopathy of (18), wherein the hepatopathy is autoimmune hapatitis.
(21) The therapeutic agent for hepatopathy of any of (18) to (20), wherein said inhibitor inhibits necrosis of hepatocytes.
(22) A method for treatment of diseases, characterized in administering the therapeutic agent of any of (15) to (17) to a patient with disease caused by activation of immunocompetent cells.
(23) A method for treatment of hepatopathy, characterized in administering the therapeutic agent for hepatopathy of any of (18) to (21) to a patient with hepatopathy.

### BRIEF DESCRIPTION OF THE DRAWINGS

Fig. 1 is a graph showing an ALT value in serum.
Fig. 2 is views showing HE-stained hepatitis tissues.
Fig. 3 is a graph showing a ratio of the number of necrosed cells in a hepatitis tissue in the microscopic view thereof.
Fig. 4 is a graph showing the amount of OPN expression in the liver of hepatitis model mice.
Fig. 5 is a view showing the result of Western blotting of hepatitis model mice. The arrow indicates a thrombin-cleaved OPN.
Fig. 6 is a graph showing the serum ALT of wild mice and OPN-deficient mice after ConA administration.
Fig. 7 is a graph showing the survival rate of wild mice and OPN-deficient mice after ConA administration.
Fig. 8 is views showing the result of intracellular cytokine staining of hepatitis model mice. (The left view shows the result of staining of the lymphocytes in the leucocytes in the liver with an anti-NK1.1 antibody and a TCR antibody. The center view (R1) shows the result of FACS analysis of OPN expression in an NK1.1⁺TCR⁺ NKT cell group fraction; and the right view (R2) shows the result thereof in an NK1.1⁻TCR⁺ T cell group fraction.
Fig. 9 is a graph showing the result of ELISA determination of the OPN amount in the culture supernatant of NKT cells and T cells separated from the liver of hepatitis model mice.
Fig. 10 is a view showing the result of detection of OPN receptor and MIP-2 in NKT cells and T cells, for which NKT cells and T cells were separated from the liver of hepatitis model mice and the mRNA is extracted from the cells and amplified through RT-PCR.
Fig. 11 is a graph showing the result of a cell migration test for the thrombin-cleaved OPN of the liver infiltration leukocytes obtained from hepatitis model mice and wild mice.
Fig. 12 is a graph showing the result of a cell migration inhibition test for the thrombin-cleaved OPN of the liver infiltration leukocytes obtained from hepatitis model mice.
Fig. 13 is views showing the result of FACS analysis for the ratio of CD69⁺CD4⁺ T cells in the liver of hepatitis model mice in administration of an M5 antibody and a control antibody.
Fig. 14 is a graph showing the calculation of the number of activated CD4⁺ T cells from the result of FACS analysis for the number of CD69⁺CD4⁺ T cells in the liver of hepatitis model mice in administration of an M5 antibody and a control antibody.
Fig. 15 is a graph showing the calculation through FACS analysis of the number of neutrophils, CD4⁺ T cells and macrophages in the liver infiltration cells of wild mice and OPN-deficient mice in 6 hours after ConA administration.
Fig. 16 is views showing the result of immunostaining of the liver tissue of wild mice in 24 hours after ConA administration. (The left view shows the result of immunostaining with an MPO antibody; and the right view shows the result of HE staining.)
Fig. 17 is a graph showing the MPO activity in the culture supernatant of neutrophiles separated from wild mice after intraperitoneal administration of casein sodium thereto, with a full-length OPN, an N-half OPN or a C-half OPN added to the culture.
Fig. 18 is a graph showing the calculation through FACS analysis of the number of neutrophiles in the liver of wild mice in 6 hours after ConA administration.
Fig. 19 is a graph showing the time course of the MIP-2 concentration in wild mice after ConA administration.
Fig. 20 is graphs showing the result of real-time PCR quantification of the expression amount of α4 and α9 integrin mRNA in the liver of wild mice after ConA administration. (The graph with black circles indicates control antibody administration; and the graph with white circles indicates M5 antibody administration.)
Fig. 21 is a graph showing the result of ELISA analysis for the IFN-γ amount in the culture supernatant of NKT cells and T cells of a mouse liver.
Fig. 22 is a graph showing the result of quantification of the IFN-γ expression amount in the liver of M5 antibody-administered ConA hepatitis mice and control antibody-administered ConA hepatitis mice, by the use of an IFN-γ EIA Kit (manufactured by Pharmingen).
Fig. 23 is a graph showing the comparison of the ALT value in administration of 400 µg of an M5 antibody to mice before 3 hours of ConA administration thereto and at the same time of ConA administration thereto.

### BEST MODE FOR CARRYING OUT THE INVENTION

The immunocompetent cell activation inhibitor of the invention (hereinafter referred to as "inhibitor of the invention") comprises an antibody to OPN or peptide fragment thereof (hereinafter referred to as "OPN or the like") (OPN-inhibitory antibody) as the active ingredient, and is prepared by combining it with any suitable pharmaceutically-acceptable carrier component.

The OPN-inhibitory antibody of the present invention can include, but not limited to, any antibody capable of recognizing OPN or the like.

The OPN-inhibitory antibody of the present invention preferably can inhibit the binding between an integrin recognizing the site of amino acid sequence RGD and OPN or the like and the binding between an integrin recognizing the site of amino acid sequence SVVYGLR and OPN or the like, more preferably, can inhibit the binding between an integrin recognizing the site of amino acid sequence RGD, for example, αvβ1, αvβ3 or αvβ5 and OPN-a, OPN-b, OPN-c or their N-terminal fragment and the binding between an integrin recognizing the site of amino acid sequence SVVYGLR, for example α9β1, α4β1 or α4β7 and OPN-a, OPN-b, OPN-c or their N-terminal fragment.

The "N-terminal fragment" in the specification means a fragment on the N-terminal side of the fragments formed through decomposition of OPN with a protease or the like. The protease is, for example, thrombin. The SVVYGLR sequence is located between serine at position 162 and arginine at position 168 of a human OPN (Accession Number: J047765).

The method of producing the OPN-inhibitory antibody in the present invention is not limited so far as the antibody produced by the method has the above-mentioned properties. The example of the method includes, but not limited to, immunization using OPN-a, OPN-b, OPN-c, N-terminal fragments thereof, a peptide that contains an amino acid sequence of the following (A) or its corresponding sequence (hereinafter referred to as a generic term "OPN-related peptide"), as an antigen:

RGDSVVYGLR (SEQ ID: No.1)          (A)

The above-mentioned "corresponding sequence" means the SVVYGLR-corresponding sequence (the sequence SVVYGLR means the sequence from serine at position 162 to arginine at position 168 in human OPN) in OPN derived from one of other animals which is, for example, swine SVVYGLR identical to the sequence in humans, SVAYGLR in monkey, SLAVGLR in mouse and rat, bovine SVAYGLK, and SVAYRLK in rabbit.

The OPN-inhibitory antibody for humans is preferably prepared by using a peptide containing the sequence (A) as an antigen. More preferably, the OPN-inhibitory antibody is prepared for example by using as an antigen the peptide (B) containing both the two sequences in series, which starts from valine residue at position 153 and ends at serine residue at position 169 of OPN-a (Accession Number: J04765), and subsequently treating the peptide according to a general method.

VDTYDGRGDSVVYGLRS (SEQ ID: No.2) (B)

In order to increase the antigenicity, preferably, a product of the OPN-related peptide bound to a biopolymer compound is used as an antigen. Examples of the biopolymer compound to be bound to the OPN-related peptide include keyhole limpet hemocyanine (hereinafter referred to as "KLH"), oval albumin (hereinafter referred to as "OVA"), bovine serum albumin (hereinafter referred to as "BSA"), rabbit serum albumin (hereinafter referred to as "RSA") and thyroglobulin. Among them, either KLH or thyroglobulin is more preferable.

The OPN-related peptide and the biopolymer compound are bound together by known methods, for example the mix acid anhydride process (B. F. Erlanger et al., (1954) : *J. Biol. Chem. ,* 234, 1090-1094) or the activated ester process (A.E. Karu et al., (1994): *J. Agric. Food Chem.,* 42, 301-309).

The mix acid anhydride for use in the mix acid anhydride process can be recovered by subjecting the OPN-related peptide to general Schotten-Baumann reaction, which is then allowed to react with a biopolymer compound to prepare the obj ect product of the peptide-polymer bound compound. The haloformate ester for use in the mix acid anhydride process includes for example methylchloroformate, methylbromoformate, ethyl chloroformate, ethyl bromoformate, isobutyl chloroformate and the like. The ratio of the peptide, the haloformate ester and the polymer compound to be used according to the method is appropriately selected in a wide range.

Herein, the Schotten-Baumann reaction is carried out in the presence of a basic compound. The basic compound for use in the reaction includes compounds for routine use for Schotten-Baumann reaction, for example, organic bases such as triethylamine, trimethylamine, pyridine, dimethylaniline, N-methylmorpholine, diazabicyclononene (DBN), diazabicycloundecene (DBU), diazabicyclooctane (DABCO); and inorganic bases such as potassium carbonate, sodium carbonate, potassium hydrogencarbonate, sodium hydrogencarbonate.

Additionally, the reaction is generally progressed at -20°C to 100°C, preferably at 0°C to 50°C, and the reaction time is about 5 minutes to 10 hours, preferably 5 minutes to 2 hours.

The reaction between the resulting mix acid anhydride and the biopolymer compound is generally practiced at -20°C to 150°C, preferably at 0°C to 100°C, for reaction time of about from 5 minutes to 10 hours, preferably 5 minutes to 5 hours. The mix acid anhydride method is generally carried out in an solvent. The solvent includes for example any of solvents commonly used for the mix acid anhydride method, specifically including halogenated hydrocarbons such as dichloromethane, chloroform and dichloroethane; aromatic hydrocarbons such as benzene, toluene and xylene; ethers such as diethyl ether, dioxane, tetrahydrofuran and dimethoxyethane; esters such as methyl acetate and ethyl acetate; and aprotic polar solvents such as N,N-dimethylformamide, dimethylsulfoxide, and hexamethylphosphortriamide; and the like.

Alternatively, the activated ester process is generally done as follows: Dissolving first the OPN-related peptide in an organic solvent, for reaction with an N-hydroxysuccinimide in the presence of a coupling agent, an N-hydroxysuccinimide-activated ester is produced.

As the coupling agent, general coupling agents for routine use in condensation reaction can be used, including, for example, dicyclohexylcarbodiimide, carbonyldiimidazole and water-soluble carbodiimide. As the organic solvent, alternatively, for example, N,N-dimethylformamide (DMF), dimethylsulfoxide and dioxane. The molar ratio of the peptide for use in the reaction to the coupling agent such as N-hydroxysuccinimide is preferably from 1:10 to 10:1, most preferably 1:1. The reaction temperature is 0 to 50°C, preferably from 22 to 27°C; while the reaction time is 5 minutes to 24 hours, preferably from 1 to 2 hours. The reaction temperature is a temperature of the individual melting points or more to the individual boiling points or less.

After the coupling reaction, the reaction solution is added to a solution dissolving a biopolymer compound therein, for reaction. In the case where the biopolymer compound has a free amino group, for example, an acid-amide bond is formed between the amino group and the carboxyl group of the peptide. The reaction temperature is 0 to 60°C, preferably 5 to 40°C, and more preferably 22 to 27°C, while the reaction time is 5 minutes to 24 hours, preferably from 1 to 16 hours, and more preferably from 1 to 2 hours.

The reaction product between the OPN-related peptide and the biopolymer compound as generated by the method is purified by dialysis or on a desalting column and the like, to recover the product of the OPN-related peptide bound to the biopolymer compound (simply referred to as "bound product" hereinafter).

Description now follows hereinbelow about the method for preparing an antibody, using the bound product thus recovered as an antigen, and an immunoassay method using the antibody. For the preparation of the antibody, herein, known methods can be utilized, appropriately, which are described in for example Zoku Seikagaku Jikken Koza (Biochemical Experimental Lecture Series), and Men-eki Seikagaku Kenkyu Ho (Immuno-Biochemistry Research Method) (Nihon Seikagaku Gakkai hen (Japan Biochemical Association, ed.)).

In order to prepare a polyclonal antibody using the bound product in accordance with the invention, an animal is immunized with the bound product to collect the antibody from the animal.

More specifically, for example, a bound product such as an OPN-related peptide-thyroglobulin bound product is first dissolved in sodium phosphate buffer (referred to as "PBS" hereinafter), which is then mixed with the Freund's complete adjuvant or the Freund's incomplete adjuvant, or an auxiliary agent such as alum. The resulting mixture is used as the immunogen for immunization of a mammalian animal.

Any animal for routine use in the field can be used as the animal for immunization, including, for example, mouse, rat, rabbit, goat, horse. Additionally, the method for dosing the immunogen for immunization may be via any of subcutaneous injection, intraperitoneal injection, intravenous injection, and intramuscular injection. Subcutaneous injection or intraperitoneal injection is preferable. Immunization can be done once or plural times at appropriate interval, preferably plural times at interval of from one week to 5 weeks.

According to a general method, then, blood is collected from the immunized animal, from which serum is separated. By purifying the polyclonal antibody fraction, the OPN inhibitory antibody can be recovered.

According to a general method, additionally, an immune cell recovered by immunizing an animal with the bound product is fused with myeloma cell to prepare a hybridoma. By collecting an antibody from a culture of the hybridoma, the OPN-inhibitory antibody can be recovered as a monoclonal antibody.

Further, according to a general method, the above-mentioned OPN-inhibitory antibody can be modified into a chimera antibody so that the antibody could have the same constant region as that of the antibody of a human that is a subject for therapy (see European Patent Publication EP0125023) or into a humanized antibody (see European Patent Publication EP0239400 or EP0455126, International Publication WO03/027151).

Further, the OPN-inhibitory antibody thus prepared may be used in the form of Fv, Fab or F(ab')₂ by protease digestion and the like.

In the case of study for OPN-related diseases by using mouse, preferably the OPN-inhibitory antibody against mouse OPN is used and such antibody is preferably prepared by using peptide RGDSLAYGLR as an antigen.

The inhibitor of the invention as described above contains the above-mentioned OPN-inhibitory antibody as the active ingredient, and it may be combined with a pharmaceutically-acceptable carrier and formulated into a pharmaceutical composition in a general method to obtain a therapeutic agent for diseases caused by activation of immunocompetent cells (hereinafter referred to as "therapeutic agent of the invention").

The dose to a patient of the OPN-inhibitory antibody in the therapeutic agent of the invention varies, depending on the symptomatic severity and the age of the patient. As the dose of the antibody is generally 0.1 to 100 mg/kg/day, preferably from 0.5 to 80 mg/kg/day or so, it is preferable to formulate the agent containing the antibody in amount corresponding to the dose.

Examples of the formulation of the therapeutic agent of the invention are parenteral preparations such as injectable solution and eye drops. Not detracting from the effect of the invention, any other component generally usable in production of pharmaceutical compositions may be added to these. The optional component includes, for example, adsorbent, preservative, antioxidant, buffer, chelating agent, colorant, emulsifier, seasoning/flavor, hardening agent, surfactant, suspending agent, sweetener, lubricant, excipient, coating agent, fluidizer, glossing agent, isotonizer, binding agent and filler.

The immunocompetent cells that are inhibited from being activated by the inhibitor of the invention thus obtained include macrophages, T cells, NKT cells and neutrophils. Among the immunocompetent cells, the inhibitor preferably inhibits the activation of T cells, NKT cells and neutrophils.

The inhibitor of the invention favorably inhibits Fas/FasL mediated cell injury and neutrophil mediated cell injury caused by the activation of the immunocompetent cells.

The diseases that may be treated and improved by the therapeutic agent of the invention include, for example, hepatopathy, asthma, arthritis, diabetes, lupus, multiple sclerosis, arteriosclerosis and lung fibrosis. Among the diseases, the therapeutic agent of the invention favorably inhibits necrosis of hepatocytes, and therefore may be a therapeutic agent effective for hepatopathy such as viral hepatitis or drug-registant hapatitis, especially for acute hepatopathy such as autoimmune hapatitis.

Though not completely clear, the effect and the mechanism of the inhibitor and the therapeutic agent of the invention may be considered as follows: The activation of immunocompetent cells that is caused directly or indirectly by OPN or by OPN fragment cleaved by a protease or the like could be inhibited by the antibody to OPN or the like (OPN-inhibitory antibody).

The effect and the mechanism of the inhibitor will be described below as an example of hepatopathy.

NKT cells in the liver with concanavalin A (hereinafter referred to as "ConA")-induced hepatitis may produce OPN, as demonstrated in the following examples. The thus-produced OPN is cleaved with a protease or the like into OPN fragment peptides. When the cleaved OPN or the like activates the NKT cells in the autocrine manner, then the NKT cells produce IFN-γ. It is known that IFN-γ has the following effect (Hong F., et al., *J. Clin. Invest.,* 2002 Nov.; 110(10): 1503-13). When the produced IFN-γ binds to the cytokine receptor existing on the cell membrane, then a JAK-STAT signaling pathway (Janus kinase-signal transducer and activator of transcription factor signaling pathway) acts. Specifically, a tyrosine kinase, JAK kinase (Janus kinase) is activated, and the thus-activated JAK kinase phosphorylates the tyrosine residue in the region inside the receptor cells, and then subsequently activates a cytokine-inducing transcription activator factor, STAT1 (signal transducer and activator of transcription factor 1). Further, the activated STAT1 activates NKT cells and CD4⁺ T cells.

It is known that activated NKT cells produce IL-4 (Kaneko Y., et al., *J. Exp. Med.,* 2000, Jan 3; 191(1): 105-14), and the resulting IL-4 acts on NKT cells in the autocrine manner and, as a result, the NKT cells express a Fas ligand. Accordingly, the NKT cells cause Fas/FasL mediated cell injury to hepatocytes.

On the other hand, after activated by OPN or the like, the NKT cells produce MIP-2 (macrophage inflammatory protein-2). It is known that MIP-2 promotes migration and accumulation of neutrophils (Xiao YQ., et al., Biochim. Biophys. Acta., 1997 Aug. 22: 1361 (2) : 138-46), and as a result, the active oxygen produced by the neutrophils causes liver injury (neutrophil mediated cell injury).

Against this, the active ingredient of the inhibitor and the therapeutic agent of the invention, the OPN-inhibitor antibody binds to OPN or peptide fragment thereof to thereby inhibit the immunocompetent cells such as macrophages, T cells, NKT cells and neutrophiles, which are activated by OPN or the like, from being activated.

In addition, since the OPN-inhibitory antibody inactivates the immunocompetent cells as so mentioned hereinabove, it may inhibit the IFN-γ production, MIP-2 production and IL-4 production by NKT cells.

Accordingly, since the IFN-yproduction is thus inhibited, STAT1, which is activated by the IFN-γ production, may be inhibited from being activated and, in addition, NKT cells and CD4⁺ T cells which are activated by STAT1 may also be inhibited from being activated.

Further, since the IL-4 production is inhibited, NKT cells that are activated by the IFN-γ production may be inhibited from being activated and the subsequent Fas ligand expression may also be inhibited.

Accordingly, as these are inhibited from being activated, Fas/FasL mediated cell injury may be thereby inhibited.

Further, since the OPN-inhibitory antibody inhibits MIP-2 production, it may also inhibit neutrophil migration and MPO production, and may therefore inhibit the subsequent neutrophil mediated cell injury.

Furthermore, the inhibitor and the agent of the invention may inhibit necrosis of hepatocytes to be caused by Fas/FasL mediated cell injury and neutrophil mediated cell injury, and may therefore relieve and cure hepatopathy.

Similarly, also for asthma, arthritis, diabetes, lupus, multiple sclerosis, arteriosclerosis and lung fibrosis, the OPN-inhibitory antibody may inhibit the disease-related immunocompetent cells from being activated and may therefore treat and cure these diseases.

### EXAMPLES

The invention will now be described in more detail in the following Examples. But the invention is not limited to these Examples.

### Example 1:

### Production of OPN-inhibitory antibody:

A synthetic peptide (2K1 peptide) mentioned below, corresponding to the inner sequence (from V153 to S169) of human OPN, was prepared and used for immunization.

### 2K1 peptide: VDTYDGRGDSVVYGLRS (SEQ ID: No. 2)

The 2K1 peptide has RGD and SVVYGL sequences recognizing αvβ3 and α9β1 integrin receptors.

The 2K1 peptide was bound to thyroglobulin, and mice were immunized with it according to a general method. The spleen monocytes of the immunized mouse and a fusion partner, X63-Ag8-653 were subjected to polyethylene glycol-mediated cell fusion, and a hybridoma was selected according to the method described in a reference (J. Immunol., 146: 3721-3728). Briefly, the hybridoma reactive with OPN-a derived from a fixed GST/OPNa and CHO cells but seemingly not reactive with GST was selected.

A monoclonal antibody, named 2K1 was obtained from the mouse immunized with 2K1. The hybridoma generating the monoclonal antibody 2K1 was named Human Osteopontin Hybridoma 2K1, and deposited as FERM BP-7883 at the Patent Organism Depository Center, the National Institute of Advanced Industrial Science and Technology (AIST Tsukuba Central 6, 1-1-1, Higashi, Tsukuba-shi, Ibaraki 305-8566, Japan) on the date of June 20, 2001.

### Test Example 1:

### Reactivity of OPN and thrombin digestion products thereof (thrombin-cleaved OPN) with OPN-inhibitory antibody:

The binding potencies of the monoclonal antibodies 2K1 recovered above to OPN and the thrombin digestion products thereof were tested by Western blotting method. It was found that the 2K1 antibody reacts with OPN-a, OPN-b, OPN-c, and with a thrombin digestion product of OPN-a, N-half OPN. Further, the 2K1 antibody reacted not only with a non-glycosylated recombinant OPN produced by *E*. *coli* but also with a glycosylated recombinant OPN produced by CHO cells (*J. Cell. Biochem. ,* 77: 487-498, 2002).

### Example 2:

### Administration of OPN-inhibitory antibody to hepatitis model mice:

For clarifying the action of the OPN-inhibitory antibody in hepatitis, the OPN-inhibitory antibody was administered to hepatitis model mice artificially produced from wild mice by administering ConA thereto, and its effect was observed.

Since a mouse line is used in this Example, the 2K1 antibody to human OPN could not be used directly as it is. Accordingly, a synthetic peptide (M5 peptide) mentioned below, corresponding to the inner sequence (from C+V138 to R153) of mouse osteopontin which includes both SLAYGLR sequences corresponding to human OPN SVVYGLR sequence and RGD sequences, was prepared. And an M5 antibody was produced in the same manner as in Example 1, and this was used.

### M5 peptide: CVDVPNGRGDSLAYGLR (SEQ ID: No. 3)

BALB/cmice (6-weekage, female) werekeptawayfromeating overnight, and an M5 antibody was intravenously administered to it at a dose of 400 µg/mouse, three hours before the ConA administration thereto. The same dose of a rabbit IgG was administered to the mice of a control group. Next, ConA was intravenously administered to them at a dose of 300 µg/mouse. 2, 6, 12 and 24 hours after the M5 antibody administration, the serum was collected and the ALT value in the serum was determined. The results are in Fig. 1. After the M5 antibody or control antibody administration, the liver of each mouse was collected 24 hours after the ConAadministration, and stained with HE. The results are in Fig. 2. The HE stained samples were quantitatively analyzed with NIH Image, and the ratio of the number of the necrosed cells in the hepatitis tissue in the microscopic view (x 40) was calculated. The results are in Fig. 3.

The ratio of the number of the necrosed cells in the hepatitis tissue (necrosis rate) was about 10 % in the liver of the mice administered with M5 antibody, but was about 50 % in the liver of the mice administered with rabbit IgG (Fig. 3). On the other hand, the ALT value of the mice administered with M5 antibody was about 1500, and was lower than the value, about 5000, of the control mice (Fig. 1). The results confirm that the M5 antibody inhibits the necrosis of hepatocytes caused by hepatitis.

As mentioned hereinabove, since the M5 antibody to mouse OPN inhibited the necrosis of hepatocytes, it is clear that the 2K1 antibody to human OPN may inhibit the necrosis of human hepatocytes caused by hepatitis.

### Example 3:

### Expression of OPN in hepatitis model mice:

Hepatitis model mice were produced by administering ConA to wild mice through their tail vein at a dose of 15 mg/kg. The OPN expression in the liver of hepatitis model mice were analyzed by an ELISA process using an mOPN ELISA kit (manufactured by Immuno-Biological Laboratories CO., LTD.) and by Western blotting process using an anti-osteopontin antibody (manufactured by Immuno-Biological Laboratories CO., LTD.). The results of the ELISA analysis are in Fig. 4. The results of the Western blotting analysis are in Fig. 5.

The ELISA analysis confirmed the time-dependent increase in the OPN concentration in the liver of the ConA-administered mice. The Western blotting analysis confirmed the increase in the thrombin-cleaved OPN in the liver.

### Example 4:

### Analysis of the function of OPN in hepatitis:

ConA was administered to wild mice and OPN-deficient mice (OPN-/-) (Rittling SR. , et al. , Exp. Nephrol. , 1999, 7: 103-113) at a dose of 200 µg/mouse, and then the serum ALT in each mouse was determined in the same manner as in Example 2. In addition, the survival rate of the mice after administration of ConA thereto at a dose of 400 µg/mouse was compared with each other. The ALT data are in Fig. 6. The survival rate data are in Fig. 7.

The ALT level of the OPN-deficient mice was lower than that of the wild mice. The survival rate of the OPN-deficient mice was higher.

### Example 5:

### Analysis of OPN and its receptor-producing cells in liver:

### (1) Preparation of liver infiltration leukocytes:

Hepatitis model mice were prepared by administering 300 µg of ConA through their tail vein, and the liver was taken out of them after 6 hours, homogenized and screened through a mesh. Next, the resultant was washed with PBS, and then, for removing the hepatocytes, the washed sample was added to 33 % Percoll containing heparin (100 U/ml) and centrifuged at 2000 rpm for 15 minutes to prepare cell pellets. The resulting cell pellets were processed with a hemolytic buffer (hereinafter referred to as "HLB") (manufactured by Immuno-Biological Laboratories CO., LTD.) for hemolyzation. Next, the processed sample was washed three times with PBS, and then added to D-MEM/10 % FCS to prepare liver infiltration leukocytes.

### (2) Detection of intracellular OPN by using liver infiltration leukocytes:

To confirm from which of NKT cells or T cells in the liver OPN is generated, intracellular cytokine staining and ELISA analysis of the supernatant of cultured cells were carried out according to the process mentioned below.

First, the liver infiltration leukocytes prepared in the above (1) were incubated in the presence of monensin for 90 minutes, and the cell surface molecules were stained with an anti-NK1.1 antibody and an anti-TCRβ antibody (both manufactured by PharMingen). Next, this was fixed with 4 % paraformaldehyde for 10 minutes, and then subjected to membrane transportation with a saponin buffer (PBS solution containing 1 % FCS, 0.1 % saponin, 0.1 % NaN₃). Further, the resultant sample was analyzed by FACS caliber (FACS caliber, manufactured by BD), using a biotinated OPN-inhibitory antibody (manufactured by Immuno-Biological Laboratories CO., LTD.) and streptoavidin-APC (manufactured by Jackson) as a secondary antibody.

An mOPN EIA kit (manufactured by Immuno-Biochemical Laboratories CO. , LTD.) was used for the ELISA analysis of the supernatant of cultured cells.

The results of the intracellular cytokine staining of hepatitis model mice are shown in Fig. 8. The data of ELISA analysis of the supernatant of cultured cells are in Fig. 9.

The intracellular cytokine staining confirmed the expression of OPN inside the cells of the NK1.1⁺TCR⁺, NKT cell group. In the ELISA analysis for OPN in the supernatant of cultured cells, the OPN expression was detected in the NK1.1⁺TCR⁺, NKT cell group, but was not in the NK1.1⁻TCR⁺, T cell group.

Accordingly, this suggests that the OPN-producing cells in the liver are principally NKT cells.

### (3) Analysis of OPN receptor and MIP-2, which relate to onset of hepatitis:

This is to confirm the presence or absence of the expression of α4 integrin, α9 integrin and MIP-2 in the NKT cells and the T cells of the liver of hepatitis model mice. RNA was extracted from the NKT cells and the T cells, and subj ected to RT-PCR. The results are given in Fig. 10. G3PDH is used as control. The primers and their sequences used in RT-PCR are shown below (SEQ ID: Nos. 4 to 11).

### G3PDH:

5'-ACCACAGTCCATGCCATCAC-3' (sense)

5'-TCCACCACCCCTGTTGCTGTA-3' (antisense)

### α4 Integrin:

5'-TGGAAGCTACTTAGGCTACT-3' (sense)

5'-TCCCACGACTTCGGTAGTAT-3' (antisense)

### α9 Integrin:

5'-AAAGGCTGCAGCTGTCCCACATGGACGAAG-3' (sense)

5'-TTTAGAGAGATATTCTTCACAGCCCCCAAA-3' (antisense)

### MIP-2:

5'-GAACAAAGGCAAGGCTAACTGA-3' (sense)

5'-AACATAACAACATCTGGGCAAT-3' (antisense)

As shown in Fig. 10, α9 integrin and MIP-2 were expressed in NKT cells but were not in T cells. On the other hand, α4 integrin was expressed both in NKT cells and in T cells.

### Example 6:

### Cell migration test to thrombin-digested OPN:

### (1) Cell migration test:

Liver infiltration leukocytes were tested for cell migration to thrombin digested OPN. 10 µg/ml of full-length OPN (rOPN: non-cleaved OPN), 1 U/ml of thrombin (Thr), and 10 µg/ml of thrombin digested OPN (Thr-OPN) were separately added to 10µg/ml of the liver infiltration leukocytes of hapatitis model mice prepared in Example 5, and incubated at 37°C for 2 hours, and then tested for cell migration capability by the use of a chemotaxis chamber (24-transwell tissue culture plate by Costar, having a pore size of 5 µm). The liver infiltration leukocytes obtained from wild mice were also subjected to the same cell migration test. The results are given in Fig. 11.

As a result of the cell migration test, the cells obtained from the hepatitis model mice showed their migration capability to thrombin digested OPN, but almost not to full-length OPN (non-cleaved OPN). On the other hand, the cells collected from wild mice were not reactive to all OPNs. Next, the receptor, if any, participating to the cell migration was identified according to an inhibitory test with antibodies.

### (2) Cell migration inhibiting test:

The migration capability of the cells obtained from hepatitis model mice to 10 µg/ml of thrombin digested OPN (Thr-OPN) was inhibited by the M5 antibody obtained in Example 2, M1 antibody (O-17) (manufactured by Immuno-Biological Laboratories CO., LTD.), anti-integrin β1 (HMβ1) antibody, anti-β3 (HMβ3) antibody, anti-αv (RMV-7) antibody and anti-α4 (R1-2) antibody (all manufactured by PharMingen), at a dose of 100 µg/ml each. The results are shown in Fig. 12.

As the result of the inhibiting test, the migration capability of the cells obtained from hepatitis model mice to thrombindigestedOPN (Thr-OPN) was inhibited by the M5 antibody. In addition, the anti-integrin β1 antibody and the anti-α4 integrin antibody also inhibited the migration capability of the cells.

### Example 7:

### CD4⁺ T cell activation inhibiting test:

The M5 antibody obtained in Example 2 was subjected to a CD4⁺ T cell activation inhibiting test. Using a PE-labeled CD4 (L3T4, manufactured by PharMingen) antibody and an FITC-labeled CD69 antibody (manufactured by PharMingen), the number of the liver infiltration activated CD4⁺ T cells was analyzed with an FACS caliber (FACS caliber by BD) (this is hereinafter referred to as "FACS analysis"). For comparison, a control antibody (normal rabbit IgG) was used and tested in the same manner as herein. The results are given in Fig. 13. The number of the activated CD4⁺ T cells was calculated from the analyzed data, and shown in Fig. 14.

As a result of the analysis of the number of the activated CD4⁺ T cells, the M5 antibody administration resulted in the decrease in the ratio of the activated CD4⁺ T cells in the liver and in the decrease in the number of the cells.

### Example 8:

### Analysis of liver infiltration cells after ConA administration: (1) Calculation of the number of neutrophils, CD4⁺ T cells, and macrophages:

ConA was administered to wildmice and OPN-deficient mice. 6 hours after the administration, the number of the neutrophils, the CD4⁺ T cells and the macrophages in the liver infiltration cells of each mouse was calculated through FACS analysis. Gr1⁺CD11b⁺, CD4⁺, and F4/80⁺ fractions are considered as fractions of neutrophiles, CD4⁺ T cells and macrophages, respectively. Gr1⁺CD11b⁺, CD4⁺, and F4/80⁺ were stained as follows: 6 hours after the ConA administration, the infiltration leukocytes were purified from the liver, and reacted with a biotinated CD4 (L3T4) antibody, a Gr1 (RB6-8C5) antibody (both manufactured by PharMingen), and a biotinated F4/80 antibody (manufactured by Caltag Laboratories), and detected with a streptoavidin-FITC (manufactured by DAKO). The number of the neutrophiles, the CD4⁺ T cells and the macrophages ineachmousewas calculated from the results of the FACS analysis, and the data are shown in Fig. 15.

As a result of the FACS analysis, it was confirmed that the number of the neutrophils in the wild mice is much larger than that of the other cells. In addition, the deficiency in the neutrophils in the OPN-deficient mice was confirmed.

### (2) Analysis of neutrophil expression in hepatitis tissue by MPO (myeloperoxidase) antibody:

A liver tissue obtained from a hepatitis model mouse was embedded in paraffin, and cut into 5-µm slices, which were subjected to endogenous peroxidase treatment. Next, the slices were reacted with a primary antibody, anti-MPO antibody (manufactured by DAKO) at 4°C overnight. Next, these were reacted with a secondary antibody, ENVISION (manufactured by DAKO) for immunostaining.

The results of immunostaining are shown in Fig. 16.

As a result, accumulation of neutrophils in the necrosed hepatocytes is observed, and this confirms the correlation between onset of hepatitis and neutrophil migration.

### Example 9:

### Analysis of function of thrombin digesed OPN in hepatitis model mice:

### (1) Influence of various OPNs on the MPO activity of neutrophils:

12 % casein sodium solution was intraperitoneally administered to mice. After 6 hours, the neutrophils were collected from the abdomen, and these were stimulated by 20 µg/ml of various GST-fused OPN protein.

A full-length mouse OPN (L17 to N294) may be cleaved with thrombin at the position between R154 and S155, and gives an N-half OPN (L17 to R154) at the N-terminal side and C-half OPN (S155 to N294) at the C-terminal side. Accordingly, these were prepared as GST-fused protein according to the method mentioned below. The N-half OPN and the C-half OPN were cloned through PCR from a mouse kidney cDNA, using the following primers (SEQ ID: Nos. 12 to 15).

### Full-length mouse OPN:

5'-TAGGGATCCCTCCCGGTGAAAGTGACTGAT-3' (sense)

5'-GTCTCGAGTTAGTTGACCTCAGGAAGATGA-3' (antisense)

### N-half OPN:

Full-length OPN forward primer (sense)

5'-AACCTCGAGTTACCTCAGTCCATAAGCCAA-3' (antisense)

### C-half OPN:

5'-CAGGGATCCTCAAAGTCTAGGAGTTTCCAG-3' (sense)

### Full-length mouse OPN reverse primer (antisense)

The PCR product obtained by the use of the above-mentioned primers were digested with restriction endonucleases BamHI and XhoI, then introduced into a vector pGEX6P-1 (manufactured by Amersham), and sequenced with ABI 310 Genetic Analyzer Automatic Sequencer (manufactured by Applied Biosystems). Next, this sequence was introduced into *E*. *coli* JM109 for transformation, and a GST-fused OPN protein was purified from them in general manner.

The neutrophils were stimulated with 20 µg/ml of the above-mentioned, GST-fused OPN protein for 36 hours, and the supernatant of the culture was analyzed for the MPO (myeloperoxidase) activity. The MPO activity was quantified as follows: 50 µl of the culture supernatant was mixed with 100 µl of 3,3',5,5'-tetramethylbenzidine liquid substrate system (manufactured by Sigma), and the mixture was heated at 37°C for 30 minutes, and the absorbance of the mixture at 450 nm was determined. The results are shown in Fig. 17. All data in the drawing are those obtained by subtracting the MPO activity of a control GST.

The results confirm the ability of the N-half OPN to increase the MPO activity.

### (2) FACS analysis of infiltration of neutrophils into liver:

The neutrophils infiltrated into the liver in 6 hours after the ConA administration were determined in the presence or absence of M5 antibody administration. The leukocytes were collected from the ConA-administered liver and subjected to FACS analysis for Gr1⁺CD11b⁺ cells therein. For comparison, a control antibody (normal rabbit IgG) was used in the same analysis as herein. The results are shown in Fig. 18.

The FACS analysis confirmed that the M5 antibody administration inhibited the infiltration of neutrophils in the liver.

### (3) Determination of MIP-2 in liver:

ConA was administered to wild mice, and after 6, 12 and 24 hours, the liver MIP-2 value was determined using an MIP-2 EIA kit (manufactured by Immuno-Biological Laboratories CO., LTD.). For comparison, a control antibody (normal rabbit IgG) was used in the same analysis as herein. The results are shown in Fig. 19.

As a result of the measurement in the liver MIP-2, it was confirmed that the M5 antibody administration resulted in the decrease in the MIP-2 expression.

### (4) Analysis of the expression of OPN receptor α4, α9 integrin in the liver:

The expression of α4 integrin and α9 integrin was determined through real-time PCR using the same primers as in Example 5. The measured data were corrected with G3PDH for quantification. For comparison, a control antibody (normal rabbit IgG) was used in the same analysis as herein. The results are shown in Fig. 20.

As a result of the analysis of the OPN receptor α4, α9 integrin expression in the liver, it was confirmed that the M5 antibody administration resulted in the decrease in the α4, α9 integrin mRNA. This suggests that the infiltration of the cells of expressing α4, α9 integrin in the liver was inhibited.

### Example 10:

### Determination of cytokine concentration in the liver:

### (1) Analysis of liver IFN-γ expression cells:

IFN-γ in the culture supernatant of NKT cells and T cells separated from ConA-administered mice was determined with an IFN-γ EIA kit (manufactured by PharMingen). The results are shown in Fig. 21. The analysis of the liver IFN-γ expression cells confirmed that IFN-γ is produced principally by the NKT cells.

### (2) Determination of liver IFN-γ:

The IFN-γ expression in the liver of M5 antibody-administered ConA hepatitis mice and control antibody-administered ConA hepatitis mice was determined with an IFN-γ EIA kit (manufactured by PharMingen). The results are shown in Fig. 22.

As a result of the determination of the liver IFN-γ, it was confirmed that the M5 antibody administration resulted in significant decrease in the IFN-γ production as compared with the control antibody administration.

From the above, it is considered that OPN produced by NKT cells may act on the NKT cells to activate the cells in the autocrine manner. When OPN was inhibited by the antibody, then the IFN-γ production was thereby decreased. Accordingly, it is suggested that OPN has an important role in activation of NKT cells.

### Example 11:

### Investigation of administration time of OPN-inhibitory antibody:

This is to investigate the administration time of the OPN-inhibitory antibody. 400 µg of the M5 antibody was administered to mice 3 hours before ConA administration thereto and simultaneously with ConA administration, and the ALT level of each mouse was determined. The results are shown in Fig. 23.

As a result of the ALT determination, the M5 antibody administration 3 hours before the ConA administration showed an especially excellent hepatitis-inhibiting effect. On the other hand, even when the M5 antibody was administered simultaneously with the ConA administration, it also showed a hepatitis-inhibiting effect.

As described hereinabove, it has been clarified that NKT cells are principal OPN-producing cells in mouse hepatitis caused by ConA administration. In addition, it has also been clarified that, when the OPN-inhibitory antibody is administered, then the production of IFN-γ and MIP-2 is thereby inhibited, and, as a result, the activation of NKT cells may be thereby inhibited. Further, since the antibody may inhibit the activation of NKT cells, it may also inhibit the production of IL-4. Moreover, it has been clarified that the antibody inhibits the activation of CD4⁺ T cells and neutrophils which are associated with the above-mentioned activation, and further inhibits Fas/FasL mediated cell injury and neutrophil mediated cell injury that are associated with the activation of the cells.

Accordingly, it has been clarified that, since the antibody inhibits the activation of the cells, it may be used for treatment of diseases caused by the activation of such immunocompetent cells.

To that effect, the mouse OPN-inhibitory antibody (M5 antibody) has the effect of inhibiting the activation of NKT cells, the activation of CD4⁺ T cells and the activation of neutrophils and is therefore usable for treatment of diseases caused by the activation of immunocompetent cells, and this suggests that the human OPN-inhibitory antibody (2K1 antibody) may also be usable for treatment of those diseases.

### INDUSTRIAL APPLICABILITY

The immunocompetent cell activation inhibitor of the invention inhibits the activation of immunocompetent cells.

Accordingly, the inhibitor is useful as the active ingredient of a therapeutic agent for diseases caused by the activation of immunocompetent cells.

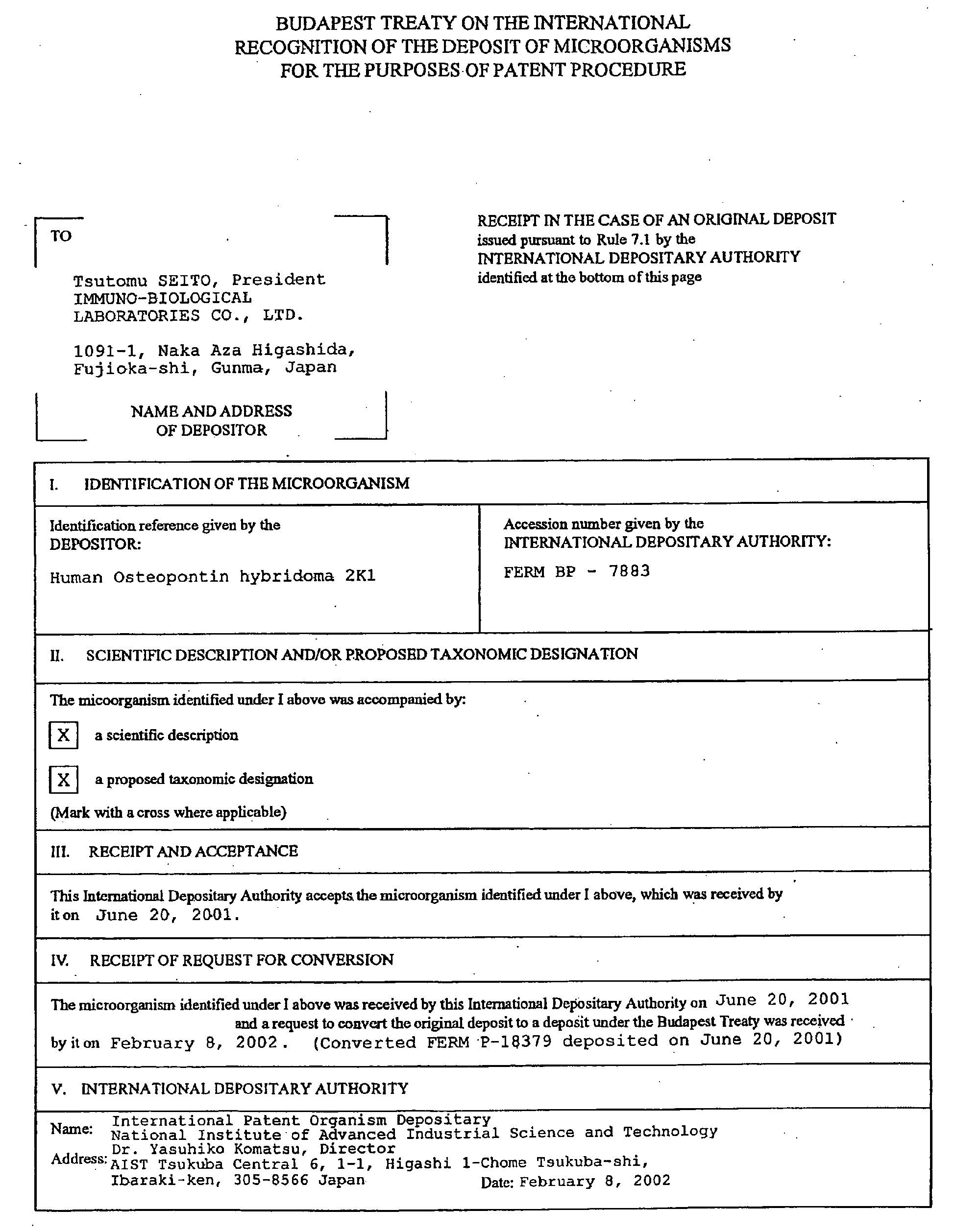

## Claims

1. An immunocompetent cell activation inhibitor comprising an antibody to osteopontin or peptide fragment thereof.

2. The immunocompetent cell activation inhibitor according to claim 1, wherein the antibody to osteopontin or peptide fragment thereof is an antibody capable of inhibiting the binding between an integrin recognizing the site of amino acid sequence RGD and osteopontin or fragment thereof, and also inhibiting the binding between an integrin recognizing the site of amino acid sequence SVVYGLR and osteopontin or fragment thereof.

3. The immunocompetent cell activation inhibitor according to claim 1; wherein the osteopontin or peptide fragment thereof is an N-terminal fragment of osteopontin.

4. The immunocompetent cell activation inhibitor according to claim 1, wherein the osteopontin or peptide fragment thereof is a peptide that contains a peptide of the following (A) :

(A)    RGDSVVYGLR

5. The immunocompetent cell activation inhibitor according to claim 1, wherein the osteopontin or peptide fragment thereof is a peptide that contains a peptide of the following (B) :

(B)    VDTYDGRGDSVVYGLRS

6. The immunocompetent cell activation inhibitor according to any one of claims 1 to 5, wherein the immunocompetent cells are NKT cells.

7. The immunocompetent cell activation inhibitor according to claim 6, wherein said inhibitor inhibits the IFN-γ production by NKT cells.

8. The immunocompetent cell activation inhibitor according to claim 6, wherein said inhibitor inhibits the MIP-2 production by NKT cells.

9. The immunocompetent cell activation inhibitor according to claim 6, wherein said inhibitor inhibits the IL-4 production by NKT cells.

10. The immunocompetent cell activation inhibitor according to any one of claims 1 to 5, wherein the immunocompetent cells are neutrophils.

11. The immunocompetent cell activation inhibitor according to any one of claims 1 to 5, wherein the immunocompetent cells are T cells.

12. The immunocompetent cell activation inhibitor according to claim 11, wherein T cells are CD4⁺ T cells.

13. The immunocompetent cell activation inhibitor according to any one of claims 1 to 12, wherein said inhibitor inhibits Fas/FasL mediated cell injury.

14. The immunocompetent cell activation inhibitor according to any one of claims 1 to 12, wherein said inhibitor inhibits neutrophil mediated cell injury.

15. A therapeutic agent for diseases caused by activation of immunocompetent cells, comprising the immunocompetent cell activation inhibitor of any one of claims 1 to 14 as the active ingredient.

16. The therapeutic agent for diseases according to claim 15, wherein the immunocompetent cells are one or more types of immunocompetent cells selected from NKT cells, neutrophils and T cells.

17. The therapeutic agent for diseases according to claim 15 or 16, wherein the diseases caused by activation of immunocompetent cells are selected from hepatitis, asthma, arthritis, diabetes, lupus, multiple sclerosis, arteriosclerosis and lung fibrosis.

18. A therapeutic agent for hepatopathy, comprising the immunocompetent cell activation inhibitor of any one of claims 1 to 14 as the active ingredient.

19. The therapeutic agent for hepatopathy according to claim 18, wherein the hepatopathy is viral hepatitis or drug-induced hapatitis.

20. The therapeutic agent for hepatopathy according to claim 18, wherein the hepatopathy is autoimmune hapatitis.

21. The therapeutic agent for hepatopathy according to any one of claims 18 to 20, wherein said inhibitor inhibits necrosis of hepatocytes.

22. A method for treatment of diseases caused by activation of immunocompetent cells, **characterized in** administering the therapeutic agent of any one of claims 15 to 17 to a patient.

23. A method for treatment of hepatopathy, **characterized in** administering the therapeutic agent for hepatopathy of any one of claims 18 to 21 to a patient.
